# FASCICULE DE BREVET EUROPEEN

(11) **EP 0 881 881 B1**
(45) Date de publication et mention de la délivrance du brevet: **09.04.2003**
(21) Numéro de dépôt: 96932673.5
(22) Date de dépôt: 27.09.1996
(51) Int. Cl.: A01N 47/02, A01N 43/56

(54) **COMPOSITION ANTIPARASITAIRE POUR LE TRAITEMENT ET LA PROTECTION DES ANIMAUX DE COMPAGNIE**
ANTIPARASITÄRE ZUSAMMENSETZUNG ZUR BEHANDLUNG UND ZUM SCHUTZ VON HAUSTIEREN
ANTIPARASITIC COMPOSITION FOR TREATING AND PROTECTING PETS

(30) Priorité: 29.09.1995 FR 9511685; 11.09.1996 FR 9611278
(43) Date de publication de la demande: 09.12.1998
(73) Titulaire: MERIAL, 69002 Lyon (FR)
(72) Inventeur: ETCHEGARAY, Jean-Pierre, F-31100 Toulouse (FR)
(74) Mandataire: Colombet, Alain André
(86) Numéro de dépôt international: FR9601521
(87) Numéro de publication internationale: WO97012521

(56) Documents cités:
- EP-A- 0 051 786
- EP-A- 0 295 117
- EP-A- 0 412 849
- EP-A- 0 500 209
- EP-A- 0 659 745
- WO-A-90/09738
- WO-A-93/06089
- WO-A-96/16544
- DE-A- 2 633 943
- DE-A- 4 414 333
- FR-A- 2 713 889
- US-A- 4 918 085
- US-A- 5 360 910
- STN International, File CABA, STN accession no. 95:207958 J.Postal et al. 'Field efficacy of a mechanical pump spray formulation XP002007096 & VETERINARY DERMATOLOGY, vol. 6, no. 3, 1995, pages 153-158,
- RESEARCH DISCLOSURE, no. 380, 1 Décembre 1995, page 802 XP000549823 "EXTENDED EFFICACY SPECTRUM OF AZOLE PESTICIDES"

## Description

La présente invention concerne une composition pour le traitement et la protection des animaux infestés ou susceptibles d'être infestés par des parasites.

Plus particulièrement, l'invention a pour but de contrôler et éliminer les parasites infestant les animaux de compagnie, et spécialement les chats et les chiens.

Les animaux de compagnie sont souvent infestés par un ou plusieurs des parasites suivants :
* les puces du chat et du chien (Ctenocephalides felis, Ctenocephalides sp. et autres),
* les tiques (Rhipicephalus sp., Ixodes sp., Dermacentor sp., Amblyomma sp. et autres)
* les galles (Demodex sp., Sarcoptes sp., Otodectes sp et autres).

Les puces causent un profond stress à l'animal et nuisent à sa santé. De plus, les puces sont également vecteurs d'agents pathogènes, tels que le taenia du chien (Dipylidium canis), et peuvent aussi s'attaquer à l'hommme.

De la même manière, les tiques peuvent aussi causer un stress à l'animal et nuire a sa santé. Elle peuvent aussi nuire à l'homme. Mais le plus grave problème des tiques est qu'elles sont le vecteur d'agents pathogènes pouvant concerner l'animal autant que l'homme. Parmi les maladies majeures devant être évitées, on peut citer les Borellioses (maladie de Lyme à Borellia burgdorferi) et les Babesioses (ou piroplasmoses à Babesia sp.), les Rickettsioses (désignées par le nom anglais de Rocky Mountain Spotted Fever). Les tiques peuvent également libérer des toxines aux propriétés paralysantes et inflammatoires, et parfois mortelles.

Enfin, la galle est particulièrement difficile à combattre car il existe très peu de matières actives agissant sur ces parasites et celles-ci réquièrent des traitements fréquents.

Il existe beaucoup d'insecticides plus ou moins actifs et plus ou moins coûteux. Mais des phénomènes de résistance sont souvent liés à leur utilisation, comme c'est le cas par exemple des carbamates, des organophosphorés et des pyréthroides.

Par ailleurs, les demandes de brevet internationale WO-A-87 03781 et européen EP-A-0 295 117 et EP-A-0500209 décrivent une grande famille de N. phénylpyrazoles ayant un très large spectre d'activité, y compris des activités antiparasitaires.

La demande de brevet internationale WO-A-96 16544 divulgue des compositions émulsionnables insecticides dont la matiere active est également un composé 1-phénylpyrazole.

L'invention a pour objectif de fournir de nouvelles compositions antiparasitaires pour le traitement et la protection des animaux, compositions qui soient d'une grande efficacité tout en étant d'utilisation facile.

Un autre objectif de l'invention est de fournir de telles compositions facilement utilisables sur tout type d'animal domestique, quelles que soient sa taille et sa nature de pelage.

Un autre objectif encore de l'invention est de fournir de telles compositions efficaces et ne demandant pas l'aspersion de tout le corps de l'animal.

Un autre objectif encore de l'invention est de fournir de telles compositions qui, appliquées localement, vont ensuite diffuser sur tout le corps de l'animal, puis sécher, tout en évitant au maximum tout phénomène de cristallisation.

Un autre objectif encore de l'invention est de fournir de telles compositions qui, après séchage, n'affectent pas l'aspect du pelage et notamment ne laissent pas de cristaux et ne rendent pas le pelage poisseux.

Ces objectifs sont atteints par l'invention, qui a pour objet des compositions antiparasitaires utiles dans le traitement et la protection des animaux domestiques infestés ou susceptibles d'être infestés par des parasites, comprenant, sous forme de solution prête à l'emploi :
a) une matière active insecticide de formule (I), 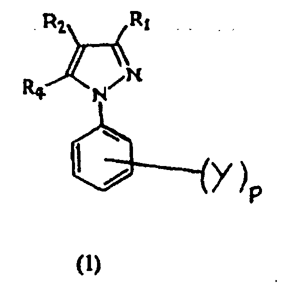 dans laquelle :
   R₁ est un atome d'halogène, CN ou méthyle ;
   R₂ est S(O)ₙR₃ ou 4,5-dicyanoimidazol 2-yle ou haloalkyl ;
   R₃ est alkyl ou haloalkyl, par exemple haloalkyl inférieur ;
   R₄ représente un atome d'hydrogène ou d'halogène ; ou un radical NR₅R₆, S(O)ₘR₇, C(O)R₇, ou C(O)OR₇, alkyl, haloalkyl ou OR₈ ou un radical 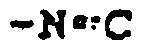 (R₉)(R₁₀) ;
   R₅ et R₆ représentent indépendamment l'atome d'hydrogène ou un radical alkyl, haloalkyl, C(O)alkyl, S(O)ᵣCF₃, acyle ou alcoxycarbonyle ; ou R₅ et R₆ peuvent former ensemble un radical alkylène divalent qui peut être interrompu par un ou deux hétéroatomes divalents, tels que l'oxygène ou le soufre;
   R₇ représente un radical alkyl ou haloalkyl ;
   R₈ représente un radical alkyl, haloalkyl ou un atome d'hydrogène ;
   R₉ représente un radical alkyl ou un atome d'hydrogène ;
   R₁₀ représente un groups phényl ou hétéroaryl éventuellement substitué par un ou plusieurs atomes d'halogène ou groupes tels que OH, -o-alkyl, -s-alkyl, cyano, ou alkyl ;
   Y représente un atome d'halogène, un radical haloalkyle ou haloalcoxy, par exemple haloalcoxy inférieur, SF₅, avec la possibilité que :
      . Y soit CN ou NO₂ aux positions 2 et 6 (par référence au carbone lié au noyau pyrazole et noté 1) ;
      . que le carbone en 2 du radical phényle soit remplacé par N ;
      . que Y soit S(O)_{q}CF₃ en position 4, mais de préférence haloalkyle, haloalcoxy ou SF₅ ;
   m, n, q, r représentent, indépendamment l'un de l'autre, un nombre entier égal à 0, 1 ou 2 ;
   p est un nombre entier égal à 1, 2, 3, 4 ou 5, de préférence égal à 1, 2 ou 3, notamment 3 ;
   sous réserve que, lorsque R₁ est méthyle, alors ou bien R₃ est haloalkyl, R₄ est NH₂, p est 2, Y en position 6 est Cl, Y en position 4 est CF₃, et le carbone en position 2 du phényle est remplacé par N ; ou bien R₂ est 4,5 dicyanoimidazol 2-yle, R₄ est Cl, p est 3, Y en position 6 est Cl, Y en position 4 est CF₃, et le carbone en position 2 du phényle est remplacé par =C-Cl,
      ce composé de formule (I) pouvant être avantageusement présent dans la formulation à raison de 1 à 20 %, de préférence de 5 à 15 % (pourcentages en poids par volume = P/V),
b) un inhibiteur de cristallisation, notamment présent à raison de 1 à 20 % (P/V), de préférence de 5 à 15 %, cet inhibiteur répondant au test selon lequel : 0,3 ml d'une solution A comprenant 10 % (P/V) du composé de formule (I) dans le solvant défini sous c) ci-après, ainsi que 10 % de cet inhibiteur, sont déposés sur une lame de verre à 20°C pendant 24 heures, à la suite de quoi on observe à l'oeil nu peu ou pas de cristaux, notamment moins de 10 cristaux, de préférence 0 cristaux, sur la lame de verre,
c) un solvant organique ayant une constante diélectrique comprise entre 10 et 35, de préférence entre 20 et 30, la teneur de ce solvant c) dans la composition globale représentant de préférence le complément à 100 % de la composition,
d) un cosolvant organique ayant un point d'ébullition inférieur à 100°C, de préférence inférieur à 80°C et ayant une constante diélectrique comprise entre 10 et 40, de préférence entre 20 et 30 ; ce cosolvant peut avantageusement être présent dans la composition selon un ratio poids/poids (P/P) de d)/c) compris entre 1/15 et 1/2. Le solvant est volatil afin de servir notamment de promoteur de séchage et est miscible à l'eau et/ou au solvant c).

De préférence, la matière active insecticide répond à la formule (II), 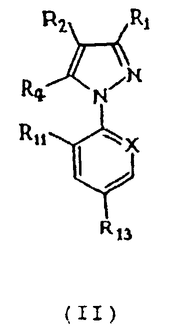 dans laquelle :
R₁ est un atome d'halogène, CN ou méthyle ;
R₂ est S(O)ₙR₃ ou 4,5-dicyanoimidazol 2-yle ou haloalkyl ;
R₃ est alkyl ou haloalkyl ;
R₄ représente un atome d'hydrogène ou d'halogène ; ou un radical NR₅R₆, S(O)ₘR₇, C(O)R₇, ou C(O)OR₇, alkyl, haloalkyl ou OR₈ ou un radical -N=C(R₉)(R₁₀) ;
R₅ et R₆ représentent indépendamment l'atome d'hydrogène ou un radical alkyl, haloalkyl, C(O)alkyl, S(O)ᵣCF₃ ou alkoxycarbonyle ; ou R₅ et R₆ peuvent former ensemble un radical alkylène divalent, tels que l'oxygène ou le soufre ;
R₇ représente un radical alkyl ou haloalkyl ;
R₈ représente un radical alkyl, haloalkyl ou un atome d'hydrogène ;
R₉ représente un radical alkyl ou un atome d'hydrogène ;
R₁₀ représente un groupe phényl ou hétéroaryl éeventuellement substitué par un ou plusieurs atomes d'halogène ou groupes tels que OH, -O-alkyl, -S-alkyl, cyano, ou alkyl ;
R₁₁ et R₁₂ représentent, indépendamment l'un de l'autre, un atome d'hydrogène ou d'halogène et éventuellement CN ou NO₂, mais H ou halogène étant préférés ;
R₁₃ représente un atome d'halogène ou un groupe haloalkyl, haloalkoxy, S(O)_{q}CF₃ ou SF₅ ;
m, n, q, r représentent, indépendamment l'un de l'autre, un nombre entier égal à 0, 1 ou 2 ;
X représente un atome d'azote trivalent ou un radical C-R₁₂, les trois autres valences de l'atome de carbone faisant partie du cycle aromatique ;
sous réserve que, lorsque R₁ est méthyle, alors ou bien R₃ est haloalkyl, R₄ est NH₂, R₁₁ est Cl, R₁₃ est CF₃, et X est N ; ou bien R₂ est 4,5 dicyanoimidazol 2-yle, R₄ est Cl, R₁₁ est Cl, R₁₃ est CF₃, et X est =C-Cl.

Les radicaux alkyle de la définition des composés de formules (I) et (II) comprennent généralement de 1 à 6 atomes de carbone. Le cycle formé par le radical alkylène divalent représentant R₅ et R₆ ainsi que l'atome d'azote auxquels R₅ et R₆ sont rattachés, est généralement un cycle à 5, 6 ou 7 chaînons.

De préférence encore, R₁ est CN, R₃ est haloalkyl, R₄ est NH₂, R₁₁ et R₁₂ sont indépendamment l'un de l'autre un atome d'halogène, R₁₃ est un haloalkyl. De préférence aussi, X est C-R₁₂.

Un composé (A) de formule (I) tout particulièrement préféré dans l'invention est le
1-[2,6-Cl₂ 4-CF₃ phényl] 3-CN 4-[SO-CF₃] 5-NH₂ pyrazole, dont la dénomination commune est fipronil.

La préparation des composés de formule (I) peut être faite selon l'un ou l'autre des procédés décrits dans les demandes de brevet WO-A-87/3781, 93/6089, 94/21606 ou européenne EP-A-295 117, ou tout autre procédé relevant de la compétence de l'homme du métier spécialiste de synthèse chimique. Pour la réalisation chimique des produits de l'invention, l'homme de l'art est considéré comme ayant à sa disposition, entre autres, tout le contenu des "Chemical Abstracts" et des documents qui y sont cités.

Quoique ceci ne soit pas préféré, la composition peut éventuellement comprendre de l'eau, notamment à raison de 0 à 30 % (volume par volume V/V), en particulier de 0 à 5 %.

La composition peut aussi comprendre un agent antioxydant destiné à inhiber l'oxydation à l'air, cet agent étant notamment présent à raison de 0,005 à 1 % (P/V), de préférence de 0,01 à 0,05 %.

Les compositions selon l'invention destinées à des animaux de compagnie notamment chiens et chats, sont généralement appliquées par dépôt cu-tané (en anglais "spot on" ou "pour on") ; il s'agit généralement d'une application localisée sur une zone de surface inférieure à 10 cm², notamment comprise entre 5 et 10 cm², en particulier en deux points et de préférence localisée entre les épaules de l'animal. Après dépôt, la composition diffuse, notamment sur tout le corps de l'animal, puis sèche, sans cristalliser ni modifier l'aspect (notamment absence de tout dépôt blanchâtre ou d'aspect poussiéreux) ni le toucher du pelage.

Les compositions selon l'invention sont particulièrement avantageuses par leur efficacité, leur rapidité d'action, ainsi que par l'aspect agréable du poil des animaux après application et séchage.

Comme solvant organique c) utilisable dans l'invention on peut citer en particulier :
l'acétone, l'acétonitrile, l'alcool benzylique, le butyldiglycol, le diméthylacétamide, le diméthylformamide, l'éther n-butylique du dipropylèneglycol, l'éthanol, l'isopropanol, le méthanol, l'éthylèneglycol monoéthyléther, l'éthylèneglycol monométhyléther, le monométhylacétamide, le monométhyléther de dipropylène glycol, les polyoxyéthylèneglycols liquides, le propylèneglycol, la 2-pyrrolidone, notamment la N-méthyl pyrrolidone, le monoéthyléther de diéthylèneglycol, l'éthylèneglycol, le diéthylphtalate, ou un mélange d'au moins deux d'entre eux.

Les solvants c) préférés sont les éthers de glycol, notamment le monoéthyléther de diéthylène glycol et le monométhyléther de dipropylèneglycol.

Comme inhibiteur de cristallisation b) utilisable dans l'invention, on peut citer en particulier :
- la polyvinylpyrrolidone, les alcools polyvinyliques, les copolymères d'acétate de vinyle et de vinylpyrrolidone, les polyéthylèneglycols, l'alcool benzylique, le mannitol, le glycérol, le sorbitol, les esters de sorbitane polyoxyéthylénés ; la lécithine, la carboxyméthylcellulose sodique ; les dérivés acryliques tels que méthacrylates et autres,
- les tensioactifs anioniques tels que les stéarates alcalins, notamment de sodium, de potassium ou d'ammonium ; le stéarate de calcium, le stéarate de triéthanolamine ; l'abiétate de sodium ; les sulfates d'alkyle, notamment le laurylsulfate de sodium et le cétylsulfate de sodium ; le dodécylbenzènesulfonate de sodium, le dioctylsulfosuccinate de sodium ; les acides gras, notamment ceux dérivés de l'huile de coprah,
- les tensioactifs cationiques tels que les sels d'ammonium quaternaires hydrosolubles de formule N⁺R'R"R'"R"",Y⁻ dans laquelle les radicaux R sont des radicaux hydrocarbonés, éventuellement hydroxylés, et Y⁻ est un anion d'un acide fort tel que les anions halogénure, sulfate et sulfonates ; le bromure de cétyltriméthylammonium fait partie des tensioactifs cationiques utilisables,
- les sels d'amine de formule N⁺R'R"R'" dans laquelle les radicaux R sont des radicaux hydrocarbonés, éventuellement hydroxylés ; le chlorhydrate d'octadécylamine fait partie des tensioactifs cationiques utilisables,
- les tensioactifs non ioniques tels que les esters de sorbitane, éventuellement polyoxyéthylénés, en particulier Polysorbate 80, les éthers d'alkyle polyoxyéthylénés ; le stéarate de polyéthylèneglycol, les dérivés polyoxyéthylénés de l'huile de ricin, les esters de polyglycérol, les alcools gras polyoxyéthylénés, les acides gras polyoxyéthylénés, les copolymères d'oxyde d'éthylène et d'oxyde de propylène,
- les tensioactifs amphotères tels que les composés lauryle substitués de la betaïne,
ou de préférence un mélange d'au moins deux d'entre eux.

De manière particulièrement préférée, on utilisera un couple inhibiteur de cristallisation, à savoir la combinaison d'un agent filmogène de type polymérique et d'un agent tensio-actif. Ces agents seront notamment choisis parmi les composés cités comme inhibiteur de cristallisation b).

Parmi les agents filmogènes de type polymérique particulièrement intéresssants, on peut citer :
- les différents grades de polyvinylpyrrolidone,
- les alcools polyvinyliques, et
- les copolymères d'acétate de vinyle et de vinylpyrrolidone.

Pour ce qui est des agents tensio-actifs, on citera tout particulièrement les tensioactifs non ioniques, de préférence les esters de sorbitane polyoxyéthylénés et notamment les différents grades de Polysorbate, par exemple le Polysorbate 80.

Agent filmogène et agent tensioactif pourront notamment être incorporés en quantités proches ou identiques dans la limite des quantités totales d'inhibiteur de cristallisation mentionnées par ailleurs.

Le couple ainsi constitué assure de manière remarquable les objectifs d'absence de cristallisation sur le poil et de maintien de l'aspect cosmétique du pelage, c'est-à-dire sans tendance au collage ou à l'aspect poisseux, malgré la forte concentration en matière active.

Comme cosolvant d), on peut citer en particulier : l'éthanol absolu, l'isopropano (propanol 2), le méthanol.

Comme agent antioxydant, on utilise notamment les agents classiques tels que : butylhydroxyanisole, butylhydroxytoluène, acide ascorbique, métabisulfite de sodium, gallate de propyle, thiosulfate de sodium, mélange d'au plus deux d'entre eux.

Les compositions selon l'invention se préparent habituellement par simple mélange des constituants tels que précédemment définis ; de manière avantageuse, on commence par mélanger la matière active dans le solvant principal, et on ajoute ensuite les autres ingrédients ou adjuvants.

La présente invention a également pour objet une méthode de traitement et/ou de protection (prévention) des animaux contre les parasites, selon laquelle on applique un volume efficace d'une composition selon l'invention sur une zone limitée de l'animal, comme cela est décrit plus haut. L'application est avantageusement effectuée en deux points et/ou dorsalement entre les épaules de l'animal.

L'objet de la méthode peut être non thérapeutique, s'agissant de nettoyer les poils et la peau des animaux en éliminant les parasites présents ainsi que leurs résidus et déjections. L'animal présente donc un pelage agréable à l'oeil et au toucher. Cela permet aussi d'éviter le développement de puces dans la maison.

L'objet peut aussi être thérapeutique lorsqu'il s'agit de traiter une parasitose ayant des conséquences pathogènes.

Le volume appliqué peut être de l'ordre de 0,3 à 1 ml, de préférence de l'ordre de 0,5 ml pour le chat, et de l'ordre de 0,3 à 3 ml pour le chien, en fonction du poids de l'animal.

Le volume appliqué de la composition correspond de préférence à une dose de composé de formule (I) comprise entre 0,3 et 60 mg, notamment entre 5 et 15 mg, par kg.

Les exemples suivants, donnés à titre non limitatif, illustrent l'invention et montrent comment elle peut être mise en oeuvre.

### Exemples 1 à 12 :

Les compositions de l'exemple 1 à 12 sont données dans le tableau suivant :

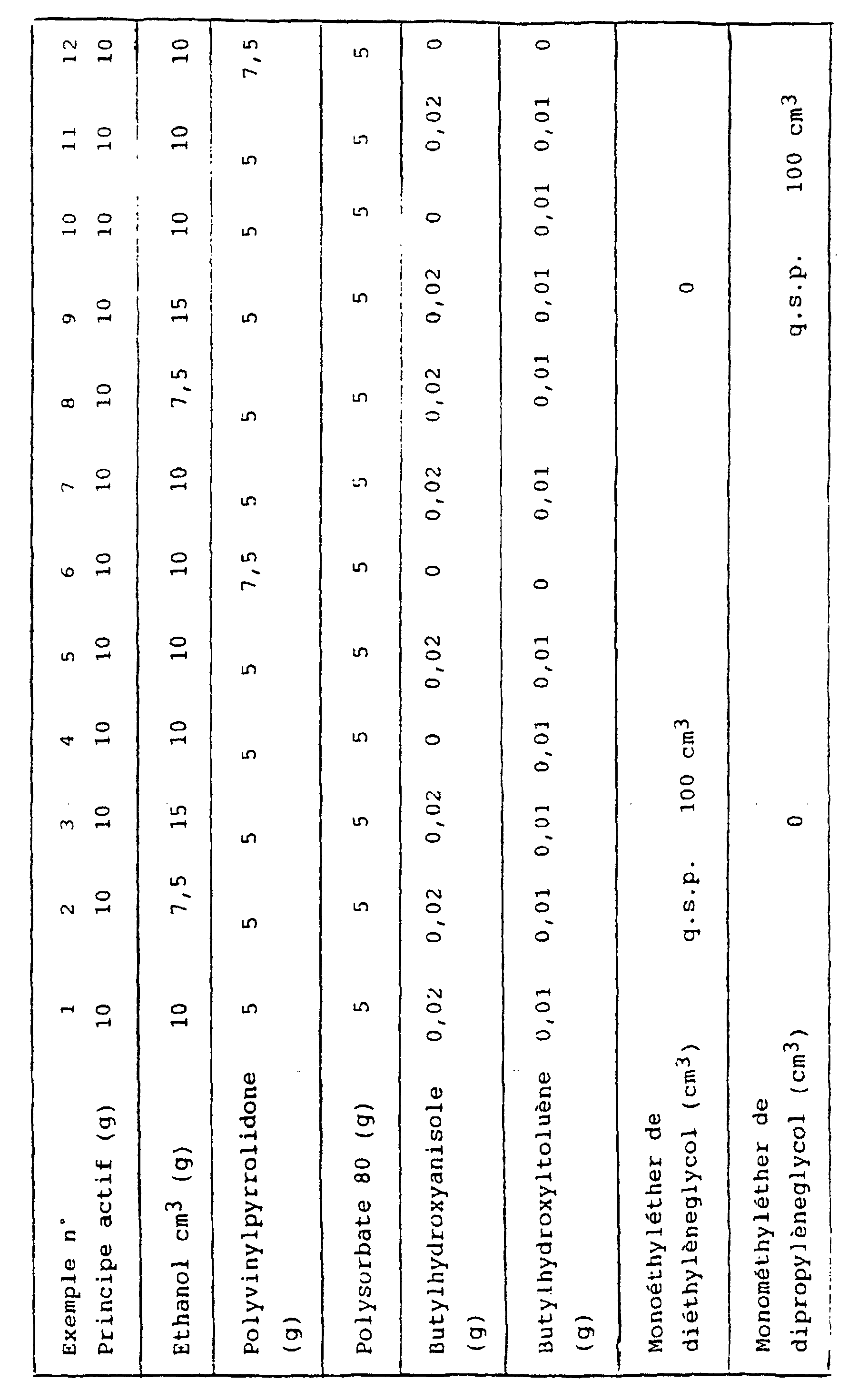

A titre d'exemple, la q.s.p. de monoéthyléther de diéthylèneglycol est d'environ 75 cm³ pour la formule de l'exemple 1.

On mélange en agitant :
10 g de principe actif 1-[4-CF₃ 2,6-Cl₂ phényl] 3-cyano 4-[CF₃-SO-] 5-NH₂ pyrazole,
totalité de l'éthanol,
60 cm³ de monoéthyléther de diéthylèneglycol ou de monométhyléther de dipropylèneglycol (solvants)
totalité de la polyvinylpyrrolidone (Kollidon® 17PF de BASF, Allemagne)
totalité du Polysorbate 80 (Tween® 80 d'ICI)
totalité du butylhydroxyanisole (si présent)
totalité du butylhydroxytoluène (si présent).

On complète à 100 cm³ avec du monoéthyléther de diéthylèneglycol ou du monométhyléther de dipropylèneglycol (pour l'exemple 1, cela correspond au volume d'environ 15 cm³ restant).

Chaque mélange constitue une solution concentrée S. 3 chiens, respectivement d'environ 7, 14 et 28 kg sont infestés avec 100 puces chacun. Deux jours après ils sont traités par application cutanée d'une solution S à raison de 0,1 ml/kg, sous forme localisée sur environ 5 cm² entre les épaules au niveau du garrot. Au bout de 24 heures, temps nécessaire à un séchage complet, l'aspect du pelage des chiens dans la zone du dépôt cutané et ailleurs est identique à l'aspect initial. Notamment le pelage de l'animal n'est ni collant ni poisseux lors d'un contact manuel et ce pelage ne comporte pas de touffe hérissée.

24 heures après traitement, les chiens sont peignés afin de retirer et compter les puces éventuellement présentes. Puis à intervalles hebdomadaires après traitement, les animaux sont réinfestés de la même manière que précédemment. 24 heures après chaque réinfestation expérimentale, un peignage est à nouveau réalisé pour retirer et compter les puces éventuellement encore présentes. Sur une période de 13 semaines, on a constaté un pourcentage de réduction de la population de puces se maintenant au-dessus de 95 % par rapport à un groupe témoin n'ayant pas reçu le traitement selon l'invention.

### Exemples 12 à 24 :

Pour les exemples 12 à 24, il suffit de remplacer, dans le tableau précédent, les exemples 1 à 12 par 12 à 24 respectivement, avec 12,5 g de principe actif. Les quantités des autres constituants ne changent pas, mise à part la quantité de solvant nécessaire pour le q.s.p. à 100 cm^{3.}

Par simple agitation, on procède au mélange des ingrédients suivants :
12,5 g du composé de l'exemple 1
totalité de l'éthanol
60 cm³ de monoéthyléther de diéthylèneglycol ou de monométhyléther de dipropylèneglycol
totalité de la polyvinylpyrrolidone.
totalité du Polysorbate 80
totalité du butylhydroxyanisole (si présent)
totalité du butylhydroxytoluène (si présent).

On complète à 100 cm³ avec du monoéthyléther de diéthylèneglycol ou de momométhyléther de dipropylèneglycol.

Utilisé dans les conditions décrites à l'exemple 1, ces mélanges conduisent à des résultats comparables. On constate une réduction de la population de puces supérieure à 95 % en moins de 24 h par rapport au groupe témoin.

## Revendications

1. Composition utile pour le traitement et la protection des animaux domestiques infastés ou susceptibles d'être infestés par des parasites, **caractérisée en ce qu'**elle comprend, sous la forme d'une solution prête à l'emploi :
a) une matière active insecticide de formule (I), 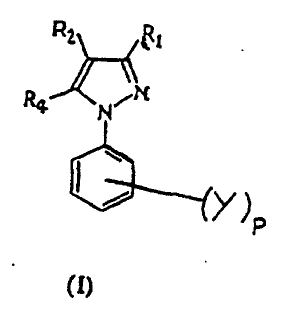 dans laquelle :
R₁ est un atome d'halogène, CN ou méthyle ;
R₂ est S(O)ₙR₃ ou 4,5-dicyanoimidazol 2-yle ou haloalkyl ;
R₃ est alkyl ou haloalkyl ;
R₄ représente un atome d'hydrogène ou d'halogène ; ou un radical NR₅R₆, S(O)ₘR₇, C(O)R₇, ou C(O)OR₇, alkyl, haloalkyl ou OR₈ ou un radical -N=C(R₉)(R₁₀) ;
R₅ et R₆ représentent indépendamment l'atome d'hydrogène ou un radical alkyl, haloalkyl, C(O)alkyl, S(O)ᵣCF₃, acyle ou alkoxycarbonyle ; ou R₅ et R₆ peuvent former ensemble un radical alkylène divalent qui peut être interrompu par un ou deux hétéroatomes divalents, tels que l'oxygène ou le soufre ;
R₇ représente un radical alkyl ou haloalkyl ;
R₈ représente un radical alkyl, haloalkyl ou un atome d'hydrogène ;
R₉ représente un radical alkyl ou un atome d'hydrogène ;
R₁₀ représente un groupe phényl ou hétéroaryl éventuellement substitué par un ou plusieurs atomes d'halogène ou groupes tels que OH, -O-alkyl, -S-alkyl, cyano, ou alkyl ;
Y représente un atome d'halogène, un radical haloalkyle ou haloalcoxy, par exemple haloalcoxy inférieur, SF₅, avec la possibilité que :
. Y soit CN ou NO₂ aux positions 2 et 6 ;
. que le carbone en 2 du radical phényle soit remplacé par N ;
. que Y soit S(O)_{q}CF₃ en position 4, mais de préférence haloalkyle, haloalcoxy ou SF₅ ;
m, n, q, r représentent, indépendamment l'un de l'autre, un nombre entier égal à 0, 1 ou 2 ;
p est un nombre entier égal à 1, 2, 3, 4 ou 5, de préférence égal à 1, 2 ou 3, notamment 3 ;
sous réserve que, lorsque R₁ est méthyle, alors ou bien R₃ est haloalkyl, R₄ est NH₂, p est 2, Y en position 6 est Cl, Y en position 4 est CF₃, et le carbone en position 2 du phényle est remplacé par N ; ou bien R₂ est 4,5 dicyanoimidazol 2-yle, R₄ est Cl, p est 3, Y en position 6 est Cl, Y en position 4 est CF₃, et le carbone en position 2 du phényle est remplacé par =C-Cl,
b) un inhibiteur de cristallisation, qui répond au test selon lequel :
0,3 ml d'une solution A comprenant 10 % (P/V) du composé de formule (I) dans le solvant défini sous c) ci-après, ainsi que 10 % de cet inhibiteur, sont déposés sur une lame de verre à 20°C pendant 24 heures, à la suite de quoi on observe à l'oeil nu moins de 10 cristaux, de préférence 0 cristaux, sur la lame de verro,
c) un solvant organique syant une constante diélectrique comprise entre 10 et 35, de préférence entre 20 et 30,
d) un cosolvant organique ayant un point d'ébullition inférieur à 100°C, de préférence inférieur & à 80°C, et une constante diélectrique comprise entre 10 et 40, de préférence entre 20 et 30.

2. Composition selon la revendication 1, **caractérisée en ce que** le composé de formule (I) est présent à raison de 1 à 20 % P/V, de préférence de 5 à 15 %, dans la composition.

3. Composition selon la revendication 1 ou 2, **caractérisée en ce que** l'inhibiteur de cristallisation est présent à raison de 1 à 20 % P/V, de préférence de 5 à 15 %, dans la composition.

4. Composition selon l'une des revendications 1 à 3, **caractérisée en ce que** le solvant organique représente le complément à 100 % de la composition.

5. Composition selon l'une des revendications 1 à 4, **caractérisée en ce que** le cosolvant organique est présent dans la composition à raison d'un ratio P/P cosolvant d)/solvant c) compris entre 1/15 et 1/2.

6. Composition selon l'une des revendications 1 à 5, **caractérisée en ce que** l'eau est présente à raison de 0 à 30 % V/V, de préférence de 0 à 5 %, dans la composition.

7. Composition selon l'une quelconque des revendications 1 à 6, **caractérisée en ce qu'**elle comprend un agent antioxydant.

8. Composition selon la revendication 7, **caractérisée en ce que** l'agent antioxydant est présent à raison de 0,005 à 1 % (P/V), de préférence de 0,01 à 0,05 %.

9. Composition selon l'une des revendications 1 à 8, **caractérisée en ce que** le solvant c) est choisi parmi le groupe consistant en :
acétone, acétonitrile, alcool benzylique, butyldiglycol, diméthylacétamide, diméthylformamide, éther n-butylique du dipropylèneglycol, éthanol, isopropanol, méthanol, éthylèneglycol monoéthyléther, éthylèneglycol monométhyléther, monométhylacétamide, monométhyléther de dipropylèneglycol, polyoxyéthylène-glycols liquides, propylèneglycol, 2-pyrrolidone, notamment N-méthyl pyrrolidone, monoéthyléther de diéthylèneglycol, éthylèneglycol, diéthylphtalate, et mélange d'au moins deux d'entre eux.

10. Composition selon l'une des revendications 1 à 9, **caractérisée en ce que** l'inhibiteur de cristallisation est choisi parmi le groupe consistant en :
- polyvinylpyrrolidone, alcools polyvinyliques, copolymères d'acétate de vinyle et de vinylpyrrolidone, polyéthylèneglycols, alcool benzylique, mannitol, glycérol, sorbitol, esters de sorbitane polyoxyéthylénés ; lécithine, carboxyméthylcellulose sodique ; dérivés acryliques tels que méthacrylates et autres ;
- tensioactifs anioniques tels que stéarates alcalins, notamment de sodium, de potassium ou d'ammonium ; stéarate de calcium, stéarate de triéthanolamine ; abiétate de sodium ; sulfates d'alkyle, notamment laurysulfate de sodium et cétylsulfate de sodium ; dodécylbenzènesulfonate de sodium, dioctylsulfosuccinate de sodium ; acides gras, notamment ceux dérivés de l'huile de coprah,
- tensioactifs cationiques tels que sels d'ammonium quaternaires hydrosolubles de formule N⁺R'R"R'"R"",Y⁻ dans laquelle les radicaux R sont des radicaux hydrocarbonés, éventuellement hydroxylés, et Y⁻ est un anion d'un acide fort tel que les anions halogénure, sulfate et sulfonates , bromure de cétyltriméthylammonium,
- sels d'amine de formule N⁺R'R"R'" dans laquelle les radicaux R sont des radicaux hydrocarbonés, éventuellement hydroxylés ; chlorhydrate d'octadécylamine,
- tensioactifs non ioniques tels que les esters de sorbitane, éventuellement polyoxyéthylénés, en particulier Polysorbate 80, les éthers d'alkyle polyoxyéthylénés ; stéarate de polyéthylèneglycol, dérivés polyoxyéthylénés de l'huile de ricin, esters de polyglycérol, alcools gras polyoxyéthylénés, acides gras polyoxyéthylénés, copolymères d'oxyde d'éthylène et d'oxyde de propylène,
- tensioactifs amphotéres tels que les composés lauryle substitués de la betaïne,
et de préférence mélanges d'au moins deux d'entre eux.

11. Composition selon l'une des revendications 1 à 10, **caractérisée en ce que**, comme agent inhibiteur de cristallisation, elle comprend un couple inhibiteur de cristallisation incluant un agent filmogène polymérique et un agent tensioactif.

12. Composition selon la revendication 11, **caractérisée en ce que** l'agent filmogène polymérique est choisi parmi le groupe consistant en :
- différents grades de polyvinylpyrrolidone ;
- alcools polyvinyliques ; et
- copolymères d'acétate de vinyle et de vinylpyrrolidone.

13. Composition selon la revendication 11 ou 12, **caractérisée en ce que** l'agent tensioactif est un tensioactif non ionique.

14. Composition selon la revendication 13, **caractérisée en ce que** l'agent tensioactif est un ester de sorbitane polyoxyéthyléné tel que Polysorbate.

15. Composition selon la revendication 14, **caractérisée en ce que** l'inhibiteur de cristallisation est un mélange de polyvinylpyrrolidone et de Polysorbate, de préférence de Polysorbate 80.

16. Composition selon l'une quelconque des revendications 1 à 15, **caractérisée en ce que** le solvant c) est un éther de glycol.

17. Composition selon la revendication 16, **caractérisée en ce que** le solvant c) est choisi dans le groupe consistant en monoéthyléther de diéthylèneglycol et monométhyléther de dipropylèneglycol.

18. Composition selon l'une des revendications 1 à 17, **caractérisée en ce que** le cosolvant d) est choisi parmi le groupe consistant en : éthanol absolu, isopropanol, méthanol.

19. Composition selon l'une des revendications 1 à 18, **caractérisée en ce qu'**elle comprend un agent antioxydant choisi parmi le groupe consistant en : butylhydroxyanisole, butylhydroxytoluène, acide ascorbique, métabisulfite de sodium, gallate de propyle, thiosulfate de sodium et mélange d'au plus deux d'entre eux.

20. Composition selon l'une quelconque des revendications 1 à 19, **caractérisée en ce que** la matière insecticide répond à la formule (II) : 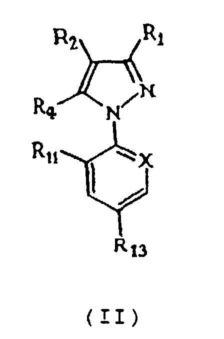 dans laquelle :
R₁ est un atome d'halogène, CN ou méthyle ;
R₂ est S(O)ₙR₃ ou 4,5-dicyanoimidazol 2-yle ou haloalkyl ;
R₃ est alkyl ou haloalkyl ;
R₄ représente un atome d'hydrogène ou d'halogène ; ou un radical NR₅R₆, S(O)ₘR₇, C(O)R₇, ou C(O)OR₇, alkyl, haloalkyl ou OR₈ ou un radical -N=C(R₉)(R₁₀) ;
R₅ et R₆ représentent indépendamment l'atome d'hydrogène ou un radical alkyl, haloalkyl, C(O)alkyl, S(O)ᵣCF₃ ou alkoxycarbonyle ; ou R₅ et R₆ peuvent former ensemble un radical alkylène divalent qui peut être interrompu par un ou deux hétéroatomes divalents, tels que l'oxygène ou le soufre ;
R₇ représente un radical alkyl ou haloalkyl ;
R₈ représente un radical alkyl, haloalkyl ou un atome d'hydrogène ;
R₉ représente un radical alkyl ou un atome d'hydrogène ;
R₁₀ représente un groupe phényl ou hétéroaryl éventuellement substitué par un ou plusieurs atomes d'halogène ou groupes tels que OH, -O-alkyl, -S-alkyl, cyano, ou alkyl ;
R₁₁ et R₁₂ représentent, indépendamment l'un de l'autre, un atome d'hydrogène ou d'halogène et éventuellement CN ou NO₂, mais H ou halogène étant préférés ;
R₁₃ représente un atome d'halogène ou un groupe haloalkyl, haloalkoxy, S(O)_{q}CF₃ ou SF₅ ;
m, n, q, r représentent, indépendamment l'un de l'autre, un nombre entier égal à 0, 1 ou 2 ;
X représente un atome d'azote trivalent ou un radical C-R₁₂, les trois autres valences de l'atome de carbone faisant partie du cycle aromatique ;
sous réserve que, lorsque R₁ est méthyle, alors ou bien R₃ est haloalkyl, R₄ est NH₂, R₁₁ est Cl, R₁₃ est CF₃, et X est N ; ou bien R₂ est 4,5 dicyanoimidazol 2-yle, R₄ est Cl, R₁₁ est Cl, R₁₃ est CF₃, et X est =C-Cl,

21. Composition selon l'une quelconque des revendications 1 à 20, **caractérisée en ce que** le composé de formule (I) ou (II) est le 1-[4-CF₃ 2,6-Cl₂ phényl] 3-cyano 4-[CF₃-SO] 5-NH₂ pyrazole.

## Claims

1. Composition useful for treating and protecting pets infested or likely to be infested with parasites, **characterised in that** it comprises, in the form of a ready-to-use solution:
a) an active insecticidal material with the formula (I) 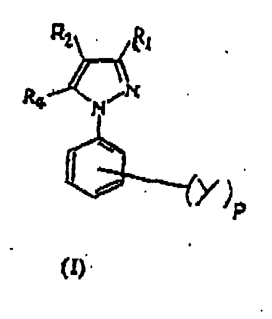 in which:
R₁ is an atom of halogen, CN or methyl;
R₂ is S(O)ₙ R₃ or 4,5-dicyano-imidazole 2-yl or haloalkyl;
R₃ is alkyl or haloalkyl;
R₄ represents a hydrogen or halogen atom; or a radical NR₅R₆ , S(O)ₘR₇, C(O)R₇ , or C(O)OR₇, alkyl, haloalkyl or OR₉ with a radical -N=C(R₉)(R₁₀);
R₅ and R₆ represent independently the hydrogen atom, of an alkyl, haloalkyl, C(O)alkyl, S(O)ᵣCF₃, acyl or alkoxy-carbonyl radical; or R₅ and R₆ can together form a divalent alkylene radical which can be interrupted by one or two divalent hetero-atoms such as oxygen or sulphur;
R₇ represents an alkyl or haloalkyl radical;
R₈ represents an alkyl, haloalkyl radical or a hydrogen atom;
R₉ represents an alkyl radical or a hydrogen atom;
R₁₀ represents a phenyl or hetero-aryl group possibly substituted by one or more halogen atoms or groups such as CH, -O-alkyl, -S-alkyl, cyano or alkyl;
Y represents a halogen atom, a haloalkyl or haloalcoxy radical, for example lower halcalcoxy, SF₅, with the possibility that:
- Y is CN or NO₂ in positions 2 and 6;
- the carbon in 2 of the phenyl radical is replaced by N;
- Y is S(O)_{q}CF₃ in position 4, but preferably haloalkyl, haloalcoxy or SF₅;
m, n, q, r represent, independently of each other, a whole number equal to 0, 1 or 2;
p is a whole number equal to 1, 2, 3, 4 or 5, preferably equal to 1, 2 or 3, particularly 3;
with the reservation that, when R₁ is methyl, then R₃ is haloalkyl, R₄ is NH₂, p is 2, Y in position 6 is Cl, Y in position 4 is CF₃, and the carbon in position 2 of the phenyl is replaced by N; or else R₂ is 4,5 dicyano imidazole 2-yl, R₄ is Cl, p is 3, Y in position 6 is Cl, Y in position 4 is _{CF33}, and the carbon in position 2 of the phenyl is replaced by =C-Cl;
b) a crystallisation inhibitor which meets the test whereby:
0.3 ml of a solution A comprising 10% (weight:volume) of the compound in formula (I) in the solvent defined under a) below, as well as 10% of this inhibitor, are deposited on a glass plate at 20°C for 24 hours, following which fewer than 10 crystals, and preferably no crystals, are observed with the naked eye on the glass plate;
c) an organic solvent with a dielectric constant between 10 and 35, preferably between 20 and 30;
d) an organic cosolvent with a boiling point below 100°C, preferably below 80°C, and a dielectric constant between 10 and 40, preferably between 20 and 30;

2. Composition according to Claim I, **characterised in that** the compound in formula (I) is present at the rate of I to 20% weight:volume, preferably from 5 to 15%, in the composition.

3. Composition according to Claim 1 or 2, **characterised in that** the crystallisation inhibitor is present at the rate of 1 to 20% weight:volume, preferably from 5 to 15%, in the composition.

4. Composition according to one of the Claims 1 to 3, **characterised in that** the organic solvent represents the complement to 100% of the composition.

5. Composition according to one of the Claims 1 to 4 **characterised in that** the organic cosolvent is present in the composition at the rate of a weight:weight cosolvent d):solvent c) ratio between 1:15 and 1:2 inclusive.

6. Composition according to one of the Claims 1 to 5, **characterised in that** the water is present at the rate of 0 to 30% volume:volume, preferably from 0 to 5%, in the composition.

7. Composition according to any one of the Claims I to 6, **characterised in that** it includes an anti-oxidising agent.

8. Composition according to Claim 7, **characterised in that** the anti-oxidising agent is present at the rate of 0.005 to 1% (weight:volume), preferably from 0.01 to 0.05%.

9. Composition according to one of the Claims 1 to 8, **characterised in that** the solvent c) is selected from among the group consisting of:
acetone, acetonitrile, benzylic alcohol, butyl diglycol, dimethyl acetamide, dimethyl formamide, n-butyl ether of dipropylene glycol, ethanol, isopropanol, methanol, ethylene glycol, monoethyl ether, ethylene glycol monomethyl ether, monomethyl acetamide, monomethyl ether of dipropylene glycol, liquid polyoxyethylene glycols, propylene glycol, 2-pyrrolidone,
particularly N-methyl pyrrolidone, diethylene glycol monoethyl ether, ethylene glycol, diethyl phthalate, and a mixture of at least two of them.

10. Composition according to one of the Claims 1 to 9, **characterised in that** the crystallisation inhibitor is selected from among the group consisting of:
- polyvinyl pyrrolidone, polyvinyl alcohols, copolymers of vinyl acetate and of vinyl pyrrolidone, polyethylene glycols, benzyl alcohol, mannitol, glycerol, sorbitol, polyoxy-ethylene sorbitan esters; lecithin, sodium carboxy-methyl cellulose; acrylic derivatives such as methacrylates and others;
- anionic surface-tension agents such as alkaline stearates, particularly of sodium, potassium or ammonium; calcium stearate, triethanolamine stearate; sodium abietate; alkyl sulphates, particularly sodium lauryl sulphate and sodium cetyl sulphate; sodium dodecyl benzene sulphonate; sodium dioctyl sulpho-succinate; fatty acids, particularly those derived from coconut oil;
- cationic surface tension agents such as hydrosoluble quaternary ammonium salts with the formula N⁺R'R"R'''R"",Y⁻ in which the R radicals are hydrocarbon radicals, possibly hydroxyls, and Y⁻ is an anion of a strong acid such as the halogenide, sulphate anions and sulfonates, cetyl trimethyl ammonium bromide;
- amino salts with the formula N⁺ R'"R"R'" in which the R radicals are hydrocarbon radicals, possibly hydroxyls; octadecyl amine chlorohydrate;
- non ionic surface tension agents such as esters of sorbitan, possibly polyoxy-ethylenated, in particular Polysorbate 80, the ethers of polyoxy-ethylene alkyl; polyethylene glycol stearate, polyoxy-ethylene derivatives of castor oil, polyglycerol esters, fatty polyoxy-ethylene alcohols, fatty polyoxy-ethylene acids, copolymers of ethylene oxide and of propylene oxide;
- amphoteric surface tension agents such as the substituted lauryl compounds of betaine, and preferably mixtures of at least two of them.

11. Composiiton according to one of the Claims 1 to 10, **characterised in that**, as the crystallisation inhibitor agent, it includes a crystallisation inhibitor pair including a polymeric film-creating agent and a surface tension agent.

12. Composition according to Claim 11, **characterised in that** the polymeric film-creating agent is chosen from among the group consisting of:
- different grades of polyvinyl pyrrolidone;
- polyvinyl alcohols; and
- copolymers of vinyl acetate and of vinyl pyrrolidone.

13. Composition according to Claim 11 or 12, **characterised in that** the surface tension agent is a non ionic surface tension agent.

14. Composition according to Claim 13, **characterised in that** the surface tension agent is an ester of polyoxy-ethylene sorbitan such as Polysorbate.

15. Composition according to Claim 14, **characterised in that** the crystallisation inhibitor is a mixture of polyvinyl pyrrolidone and Polysorbate, preferably Polysorbate 80.

16. Composition according to any one of the Claims 1 to 15, **characterised in that** the solvent c) is a glycol ether.

17. Composition according to Claim 16, **characterised in that** the solvent c) is selected from the group consisting of diethylene glycol monoethyl ether and dipropylene glycol monoethyl ether.

18. Composition according to one of the Claims 1 to 17, **characterised in that** the cosolvent d) is selected from among the group consisting of absolute ethanol, isopropanol, methanol.

19. Composition according to one of the Claims 1 to 18, **characterised in that** it includes an anti-oxidising agent selected from among the group consisting of: butyl hydroxy-anisole, butyl hydroxy-toluene, ascorbic acid, sodium meta-bisulphite, propyl gallate, sodium thiosulphate and a mixture of two of them at the most

20. Composition according to any one of the Claims 1 to 19, **characterised in that** the insecticidal material complies with the formula (II): 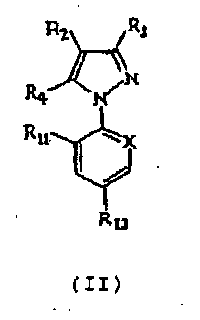 in which:
R₁ is a halogen, CN or methyl atom;
R₂ is S(O)ₙR₃ or 4,5-dicyanoimidazole 2-yl or haloalkyl;
R₃ is alkyl or haloalkyl;
R₄ represents a hydrogen or halogen atom; or an NR₅ R₆, S(O)ₘ R₇, C(O)R₇ radical, or C(O)OR₇, alkyl, haloalkyl or OR₈ or a -N=C(R₉)(R₁₀) radical;
R₅ and R₆ represent independently the hydrogen atom or an alkyl, haloalkyl, C(O)alkyl, S(O)₄ CF₃ or alkoxycarbonyl radical; or R₅ and R₆ can together form a divalent alkylene radical which can be interrupted by one or two divalent hetero-atoms such as oxygen or sulphur;
R₇ represents an alkyl or haloalkyl radical;
R₈ represents an alkyl, haloalkyl radical or a hydrogen atom;
R₉ represents an alkyl radical or a hydrogen atom;
R₁₀ represents a phenyl or hetero-aryl group, possibly substituted by one or more halogen atoms or groups such as OH, -O-alkyl, -S-alkyl, cyano, or alkyl;
R₁₁ and R₁₂ represent, independently of each other, an atom of hydrogen or of halogen and possibly CN or NO₂, but H or halogen are preferred;
R₁₃ represents a halogen atom or a haloalkyl group, haloalkoxy, S(O)_{q} CF₃ or SF₅;
m, n, q, r represent, independently of each other, a whole number equal to 0, 1 or 2;
X represents a trivalent nitrogen atom or a C-R₁₂ radical, the other three valences of the carbon atom forming part of the aromatic cycle;
with the reservation that, when R₁ is methyl, when or in any case R₃ is haloalkyl, R₄ is NH₂, R₁₁ is Cl, R₁₃ is CF₃, and X is N; or in any case R₂ is 4,5 dicyano imidazole 2-yl, R₄ is Cl, R₁₁ is Cl, R₁₃ is CF₃, and X is =C-Cl;

21. Composition according to any one of the Claims 1 to 20, **characterised in that** the compound in formula (I) or (II) is 1-[4-CF₃ 2,6-Cl₂ phenyl] 3-cyano 4-[CF₃-SO) 5-NH₂ pyrazole.

## Patentansprüche

1. Zusammensetzung, die geeignet ist für die Behandlung und den Schutz von Haustieren, die von Parasiten befallen sind oder leicht von Parasiten befallen werden, dadurch charakterisiert, dass sie in Form einer gebrauchsfertigen Lösung umfasst:
a) einen insektizid wirksamen Bestandteil der Formel (I) 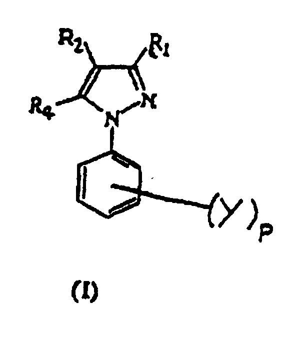 wobel:
R₁ ein Halogenatom, CN oder Methyl ist;
R₂ S(O)ₙR₃ oder 4,5-Dicyanoimidazol-2-yl oder Haloalkyl ist;
R₃ Alkyl oder Haloalkyl ist;
R₄ ein Wasserstoffatom oder ein Halogenatom darstellt; oder einen Rest NR₅R₆, S(O)ₘR₇, C(O)R₇ oder C(O)OR₇, Alkyl, Haloalkyl oder OR₈ oder einen Rest -N=C(R₉)(R₁₀);
R₅ und R₆ unabhängig ein Wasserstoffatom oder einen Alkyl-, Haloalkyl-, C(O)Alkyl-, S(O)ᵣCF₃-, Acyl- oder Alkoxycarbonylrest darstellen; oder R₅ und R₆ gemeinsam einen divalenten Alkylenrest bilden können, der durch ein oder mehr divalente Heteroatome wie Sauerstoff oder Schwefel unterbrochen sein kann;
R₇ einen Alkyl- oder Haloalkylrest darstellt;
R₈ einen Alkyl-, Hatoalkylrest oder ein Wasserstoffatom darstellt;
R₉ einen Alkylrest oder ein Wasserstoffatom darstellt;
R₁₀ eine Phenyl- oder Heteroarylgruppe darstellt, gegebenenfalls substituiert durch ein oder mehr Halogenatome oder Gruppen wie OH, -O-Alkyl, -S-Alkyl, Cyano, oder Alkyl;
Y ein Halogenatom, einen Haloalkyl- oder Haloalkoxyrest darstellt, beispielsweise niedriges Haloalkoxy, SF₅, mit der Möglichkeit, dass:
Y in den Positionen 2 und 6 CN oder NO₂ ist;
dass das Kohlenstoffatom an Position 2 des Phenylrestes durch N ersetzt ist;
dass Y S(O)_{q}CF₃ in der Position 4 ist, jedoch bevorzugt Haloalkyl, Haloaikoxy oder SF₅;
m, n, q, r unabhängig voneinander eine ganze Zahl gleich 0, 1 oder 2 darstellen;
p eine ganze Zahl gleich 1, 2, 3, 4 oder 5 darstellt, bevorzugt gleich 1, 2 oder 3, insbesondere 3;
vorausgesetzt dass, wenn R, Methyl ist, dann R₃ Haloalkyl ist, R₄ NH₂ ist, p 2 ist, y in Position 6 Cl ist, Y in Position 4 CF₃ ist, und das Kohlenstoffatom an Position 2 des Phenyl durch N ersetzt ist; oder aber R₂ 4,5-Dicyanoimidazol-2-yl ist, R₄ Cl ist, p 3 ist, Y in Position 6 Cl ist, Y In Position 4 CF₃ ist, und das Kohlenstoffatom an Position 2 des Phenyl durch -C-Cl ersetzt ist,
b) einen Kristallisationsinhibitor, der einem folgenden Test genügt:
0,3 ml einer Lösung A, umfassend 10 % (m/v) der Verbindung der Formel (I) im unter c) definierten Lösungsmittel und 10 % dieses Inhibitors, werden während 24 Stunden auf einer Glasplatte bei 20°C gegeben, und anschließend beobachtet man mit dem bloßen Auge höchstens 10 Kristalle, bevorzugt 0 Kristalle auf der Glasplatte,
c) ein organisches Lösungsmittel, das eine Dielektrizitätskonstante zwischen 10 und 35, bevorzugt zwischen 20 und 30 aufweist,
d) ein organisches Co-Solvens, das einen Siedepunkt von weniger als 100°C, bevorzugt weniger als 80°C, und eine Dielektrizitätskonstante zwischen 10 und 40, bevorzugt zwischen 20 und 30 aufweist.

2. Zusammensetzung nach Anspruch 1, dadurch charakterisiert, dass die Verbindung der Formel (I) in einer Menge von 1 bis 20 % m/v, bevorzugt 5 bis 15 % in der Zusammensetzung vorliegt.

3. Zusammensetzung nach Anspruch 1 oder 2, dadurch charakterisiert, dass der Kristallisationsinhibitor in einer Menge von 1 bis 20 % m/v, bevorzugt 5 bis 15 % in der Zusammensetzung vorliegt.

4. Zusammensetzung nach einem der Ansprüche 1 bis 3, dadurch charakterisiert, dass das organische Lösungsmittel den Rest auf 100 % der Zusammensetzung darstellt.

5. Zusammensetzung nach einem der Ansprüche 1 bis 4, dadurch charakterisiert, dass das organische Co-Solvens in der Zusammensetzung in einem Verhältnis m/m Co-Solvens d)/Lösungsmittel c) zwischen 1/15 und 1/2 vorliegt.

6. Zusammensetzung nach einem der Ansprüche 1 bis 5, dadurch charakterisiert, dass Wasser in einer Menge von 0 bis 30 % v/v, bevorzugt 0 bis 5 % in der Zusammensetzung vorliegt.

7. Zusammensetzung nach einem der Ansprüche 1 bis 6, dadurch charakterisiert, dass sie ein Antioxidationsmittel enthält.

8. Zusammensetzung nach Anspruch 7, dadurch charakterisiert, dass das Antioxidationsmittel in einer Menge von 0,005 bis 1 % (m/v), bevorzugt 0,01 bis 0,05 %, vorliegt.

9. Zusammensetzung nach einem der Ansprüche 1 bis 8, dadurch charakterisiert, dass das Lösungsmittel c) aus der Gruppe ausgewählt wird, die besteht aus: Aceton, Acetonitril, Benzylalkohol, Butyldiglykol, Dimethylacetamid, Dimethylformamid, Dipropylenglykol-n-butylether, Ethanol, Isopropanol, Methanol, Ethylenglykolmonoethylether, Ethylenglykolmonomethylether, Monomethylacetamid, Dipropylenglykolmonomethylester, flüssigen Polyoxyethylenglykolen, Propylenglykol, 2-Pyrrolidon, insbesondere N-Methylpyrrolidon, Diethylenglykolmonoethylether, Ethylenglykol, Diethylphthalat, und Mischungen von mindestens zwel davon.

10. Zusammensetzung nach einem der Ansprüche 1 bis 9, dadurch charakterisiert, dass der Kristallisationsinhibftor ausgewählt aus der Gruppe, die besteht aus:
- Polyvinylpyrrolidon, Polyvinylalkoholen, Vinylacetat-Vinylpyrrolidon-Copolymeren, Polyethylenglykolen, Benzylalkohol, Mannitol, Glycerin, Sorbitol, Sorbitanpolyoxyethylenestern, Lecithin, Natriumcarboxymethylcellulose; AcrylDerivaten wie Methacrylat und weiteren;
- anionischen oberflächenaktiven Stoffen wie Alkalistearaten, insbesondere von Natrium, Kalium oder Ammonium; Calciumstearat, Triethanolaminstearat; Natriumabtetat; Alkylsulfaten, insbesondere Natriumlaurylsulfat und Natriumcetylsulfat; Natriumdodecylbenzolsulfonat, Natrlumdioctylsuccinat; Fettsäuren, insbesondere aus Coprahöl erhaltenen,
- kationischen oberflächenaktiven Stoffen wie wasserlöslichen quaternären Ammoniumsalzen der Formel N⁺R'R"R'''R''''Y⁻, wobei die Reste R Kohlenwasserstoffreste sind, gegebenenfalls hydroxyliert, und Y⁻ ein Anion einer starken Säure ist wie Halogenid-, Sulfat- und Sulfonatanlon, Cetyltrimethylammoniumbromid,
- Aminsalzen der Formel N⁺R'R"R''', wobei die Reste R Kohlenwasserstoffreste sind, gegebenenfalls hydroxyliert, Octadecylaminchlorohydrat,
- . nicht-ionischen oberflächenaktiven Stoffen wie Sorbitanestern, gegebenenfalls polyoxyethyliert, insbesondere Polysorbat 80, Polyoxyethylenalkylethern; Polyethylenglykolstearat, polyoxyethylierten Derivaten von Rizinusöl, Polyglycerinestern, polyoxyethylierten Fettalkoholen, polyoxyethylierten Fettsäuren, Copolymeren von Ethylenoxid und Propylenoxid,
- amphoteren oberflächenaktiven Stoffen wie mit Betain substituierten Lauryl-Verbindungen,
und bevorzugt Mischungen von mindestens zwei von ihnen.

11. Zusammensetzung nach einem der Ansprüche 1 bis 10, dadurch charakterisiert, dass sie als Kristallisationsinhlbltor ein Paar aus Kristallisationsinhibitoren umfasst, die ein filmbildendes polymeres Mittel und einen oberflächenaktiven Stoff einschließen.

12. Zusammensetzung nach Anspruch 11, dadurch charakterisiert, dass das filmbildende polymere Mittel ausgewählt wird aus der Gruppe, die besteht aus:
- verschiedenen Graden von Polyvinylpyrrolidon;
- Polyvinylalkoholen,
- Vinylacetat-Vinylpyrrolidon-Copolymeren

13. Zusammensetzung nach Anspruch 11 oder 12, dadurch charakterisiert, dass der oberflächenaktive Stoff ein nicht-ionischer oberflächenaktiver Stoff ist.

14. Zusammensetzung nach Anspruch 13, dadurch charakterisiert, dass der oberflächenaktive Stoff ein Polyoxyethyfensorbitanester wie Polysorbat ist.

15. Zusammensetzung nach Anspruch 14, dadurch charakterisiert, dass der Kristallisationsinhibitor eine Mischung von Polyvinylpyrrolidon und Polysorbat ist, bevorzugt Polysorbat 80.

16. Zusammensetzung nach einem der Ansprüche 1 bis 15, dadurch charakterisiert, dass das Lösungsmittel c) ein Glykolether ist.

17. Zusammensetzung nach Anspruch 16, dadurch charakterisiert, dass das Lösungsmittel c) ausgewählt wird aus der Gruppe, die besteht aus Diethylenglykolmonoethylether und Dipropylenglykolmonomethylether.

18. Zusammensetzung nach einem der Ansprüche 1 bis 17, dadurch charakterisiert, dass das Co-Solvens d) ausgewählt wird aus der Gruppe, die besteht aus absolutem Ethanol, Isopropanol, Methanol.

19. Zusammensetzung nach einem der Ansprüche 1 bis 18, dadurch charakterisiert, dass sie ein Antioxidationsmittel umfasst, das ausgewählt wird aus der Gruppe, welche besteht aus: Butylhydroxyanisol, Butylhydroxytoluol, Ascorbinsäure, Natriummetabisulfit, Propylgallat, Natriumthiosulfat und einer Mischung von mindestens zwei von ihnen.

20. Zusammensetzung nach einem der Ansprüche 1 bis 19, dadurch charakterisiert, dass der insektizide Stoff der Formel (II) entspricht: 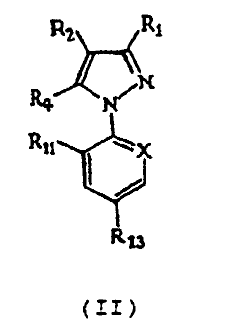 wobei
R₁ ein Halogenatom, CN oder Methyl ist;
R₂ S(O)ₙR₃ oder 4,5-Dicyanoimldazol-2-yl oder Haloalkyl ist;
R₃ Alkyl oder Haloalkyl ist;
R₄ ein Wasserstoffatom oder ein Halogenatom darstellt; oder einen Rest NR₅R₆, S(O)ₘR₇, C(O)R₇ oder C(O)OR₇, Alkyl, Haloalkyl oder OR₈ oder einen Rest -N=C(R₉)(R₁₀);
R₅ und R₆ unabhängig ein Wasserstoffatom oder einen Alkyl-, Haloalkyl-, C(O)Alkyl-, S(O)ᵣCF₃-, Acyl- oder Alkoxycarbonylrest darstellen; oder R₅ und R₆ weiter gemeinsam einen divalenten Alkylenrest bilden können, der durch ein oder mehr divalente Heteroatome wie Sauerstoff oder Schwefel unterbrochen sein kann;
R₇ einen Alkyl- oder Haloalkylrest darstellt;
R₈ einen Alkyl-, Haloalkylrest oder ein Wasserstoffatom darstellt;
R₉ einen Alkylrest oder ein Wasserstoffatom darstellt;
R₁₀ eine Phenyl- oder Heteroarytgruppe darstellt, gegebenenfalls substituiert durch ein oder mehr Halogenatome oder Gruppen wie OH, -O-Alkyl, -S-Alkyl, Cyano, oder Alkyl;
R₁₁ und R₁₂ unabhängig voneinander ein Wasserstoffatom oder ein Halogenatom darstellen und gegebenenfalls CN oder NO₂, wobei H oder Halogen jedoch bevorzugt sind;
R₁₃ ein Halogenatom oder eine Haloalkyl-, Haloalkoxy-, S(O)_{q}CF₃- oder SF₅-Gruppe darstellen;
m, n, q, r unabhängig voneinander eine ganze Zahl gleich 0, 1 oder 2 darstellen;
X ein trivalentes Stickstoffatom oder einen Rest C-R₁₂ darstellt, wobei die drei weiteren Valenzen des Kohlenstoffatoms Teil des aromatischen Rings sind;
vorausgesetzt, dass wenn R, Methyl ist, dann R₃ Haloalkyl ist, R₄ NH₂ ist, R₁₁ Cl ist, R₁₃ CF₃ ist und X N ist; oder aber R₂ 4,5-Dicyanoimidazol-2-yl ist, R₄ Cl ist, R₁₁ Cl ist, R₁₃ CF₃ ist und X =C-Cl ist.

21. Zusammensetzung nach einem der Ansprüche 1 bis 20, dadurch charakterisiert, dass die Verbindung der Formel (I) oder (II) 1-[4-CF₃-2,6-Cl₂phenyl]-3-cyano-4-[CF₃-SO]-5-NH₂-pyrazol ist.
